# EUROPEAN PATENT SPECIFICATION

(11) **EP 1 478 360 B1**
(45) Date of publication and mention of the grant of the patent: **15.04.2009**
(21) Application number: 03734774.7
(22) Date of filing: 03.02.2003
(51) Int. Cl.: A61K 31/4525, A61K 31/343, A61K 31/135, A61K 31/15, A61K 31/137, A61K 31/55, A61P 35/00, A61P 35/02

(54) **AGENTS WHICH INTERACT WITH A SEROTONIN TRANSPORTER FOR THE TREATMENT OF CANCER**
MIT EINEM SEROTONIN-TRANSPORTER ZUSAMMENWIRKENDE MITTEL ZUR KREBSBEHANDLUNG
AGENTS INTERAGISSANT AVEC UN TRANSPORTEUR DE LA SEROTONINE POUR LE TRAITEMENT DU CANCER

(30) Priority: 01.02.2002 GB 0202337
(43) Date of publication of application: 24.11.2004
(73) Proprietor: Celentyx Limited, Birmingham B15 25Q (GB)
(72) Inventor: GORDON, John, Birmingham B30 1TB (GB); BARNES, Nicholas, Mark, Kidderminster DY10 4HU (GB)
(74) Representative: Perry, Robert Edward
(86) International application number: PCT/GB2003/000436
(87) International publication number: WO 2003/063866

(56) References cited:
- EP-A- 0 727 206
- US-A- 6 054 489
- US-A- 6 162 417
- SERAFEIM ADAMANTIOS ET AL: "Selective serotonin reuptake inhibitors can stop Burkitt's lymphoma cells from committing suicide." IMMUNOLOGY, vol. 101, no. Supplement 1, December 2000 (2000-12), page 65 XP009010342 Annual Congress of the British Society for Immunology;Harrogate, UK; December 05-08, 2000 ISSN: 0019-2805
- XIA Z ET AL: "The antidepressants imipramine, clomipramine, and citalopram induce apoptosis in human acute myeloid leukemia HL-60 cells via caspase-3 activation." JOURNAL OF BIOCHEMICAL AND MOLECULAR TOXICOLOGY. UNITED STATES 1999, vol. 13, no. 6, 1999, pages 338-347, XP009010373 ISSN: 1095-6670
- TSURUO T ET AL: "Potentiation of vincristine and adriamycin effects in human hemopoietic tumor cell lines by calcium antagonists and calmodulin inhibitors" CANCER RESEARCH, AMERICAN ASSOCIATION FOR CANCER RESEARCH, BALTIMORE, MD, US, vol. 43, no. 5, May 1983 (1983-05), pages 2267-2272, XP002116183 ISSN: 0008-5472
- STEINGART ALLAN B ET AL: "Do antidepressants cause, promote, or inhibit cancers?" JOURNAL OF CLINICAL EPIDEMIOLOGY, vol. 48, no. 11, 1995, pages 1407-1412, XP002240302 ISSN: 0895-4356
- SERAFEIM ADAMANTIOS ET AL: "5-Hydroxytryptamine drives apoptosis in biopsylike Burkitt lymphoma cells: Reversal by selective serotonin reuptake inhibitors." BLOOD, vol. 99, no. 7, 1 April 2002 (2002-04-01), pages 2545-2553, XP002240303 April 1, 2002 ISSN: 0006-4971

## Description

Cancer is one of the main causes of death in the developed world. The disease takes many forms and affects almost all types of cell. Treatments for cancer are many and varied, and are often unpleasant and can have serious side effects.

Burkitt's Lymphoma (BL) is a cancer endemic to the malarial belts of equatorial Africa, North-eastern Brazil and Papua New Guinea, but whose incidence is increasing dramatically outside these endemic regions due to its association with HIV infection. Among North Americans there is a thousand fold increase in the incidence of BL among individuals with AIDS, and this trend is likely to continue as these patients live longer through the provision of better treatment regimes to manage their immune deficiency.

Burkitt's lymphoma is a highly aggressive malignancy, but is nonetheless characterised by a predisposition to exhibit a high degree of apoptosis which can be seen by its classical "starry sky" histology. This is due to the origin of the malignancy in germinal centre (GC) cells which do not express the pro-survival protein Bcl-2 and therefore have a propensity for spontaneous apoptosis, which is carried over to the BL tumour cells.

Burkitt's lymphoma is currently treated by aggressive combination chemotherapy, usually over a 6-month period and requiring frequent hospitalisations. While there is a 3-year relapse-free survival rate of approximately 80% for patients with local disease, patients with disseminated tumour respond less well to chemotherapy and have poor survival rates. The limited medical resources in regions where BL is endemic also limit survival. In individuals with AIDS, non-Hodgkin's lymphomas tend to be aggressive and advanced at diagnosis. The median survival of HIV-positive patients with non-Hodgkin's lymphomas is 6 months: Burkitt's lymphoma comprises approximately 20% of such lymphomas and is diagnosed in some 2% of AIDS patients. Intensive chemotherapy is clearly not the most desirable course of treatment for immunosuppressed individuals.

US 6, 162,417 is concerned with compounds that selectively bind the serotonin transporter primarily for PET or SPECT imaging in which case the compounds will contain at least one radioactive isotope. Reference is made to the use of the compounds for treatment but such treatments are depression, anxiety etc which are normally associated with SERT function. Reference is also made to radioactive isotopes of the compounds for the treatment of various cancers including brain cancer, breast cancer and leukaemias.

EP 0 727 206 discloses the use of sertraline for the treatment of a long list of cancers. No rationale is given for the mode of action and no supporting data is provided.

Serafeim et al ('Selective Serotonin Reuptake Inhibitors Can Stop Burkitt's Lymphoma Cells From Committing Suicide.' Immunology, Vol. 101, supplement 1, December 2000, Page 65) teach that 5-HT induces cell death in Burkitt's lymphoma cells but that this effect is blocked by SSRI's.

Xia et al 'The Antidepressants Imipramine, Clomipramine, and Citalopram Induce Apoptosis in Human Acute Myeloid Leukemia HL-60 Cells via Caspase-3 Activation.' Journal of Biochemical and Molecular Toxicology, 1999, Vol. 13, No.6, pages 338 to 347) disclose the anti-neoplastic effects of the titled compounds in human acute myeloid leukaemia HL-60 cells. The same authors report that apoptosis is induced in normal lymphocytes at higher concentrations than are effective for the HL-60 cells (Xia et al J Biochemical Toxicology, 1996, Vol 11, No. 4, pages 203-204).

Tsuruo et al (Potentiation of Vincrinstine and Adriamycin Effects in Human Hemopoietic Tumor Cell Lines by Calcium Antagonists and Calmodulin Inhibitors, Cancer Research, American Association for Cancer Research, Baltimore, Vol. 43, No. 5, May 1983, pages 2267-2272) refer to clomipramine as a calmodulin inhibitor for the potentiation of vincrinstine in certain tumor cell lines. It is specifically stated that, at the concentrations used, the calmodulin inhibitors did not induce cytotoxicity to the tumor cells.

Serotonin (5-HT) is a well known neurotransmitter of the central nervous system which has also been implicated in diverse aspects of immune regulation. Outside of the central nervous system (CNS) 5-HT is mainly produced by enterochromaffin cells of the gut and is taken up via an active transport mechanism into a number of cell types.

It is known that the action of 5-HT, on both neuronal and other cells, can be mediated either through the many different receptor subtypes to be found on the surface of the target cells, or via an active uptake mechanism that relies on the SERT (Mossner, R. Lesch, K.P., Brain Behav. Immun. (1998) 12: 249-271. Schorr, E.C. Arnason, G.W. Brain Behav. Immun. (1999) 13: 271-278. Barnes, N.M. Sharp, T. Neuropharmacology. (1999) 38: 1083-1152).

The SERT is expressed on cells outside of the CNS although the extent of its distribution in the periphery is not fully established. Platelets are by far the best-studied cell for characteristics of peripheral SERT but other haemopoietic cells, particularly those of the immune system, also carry the SERT. These include B lymphocytes, T lymphocytes, and macrophages. The rationale for these cells to carry functional SERT is not known.

It has been suggested that the 5-HT uptake activity of SERT in the CNS, whilst a normal and necessary process, may be involved in neuronal cell death, particularly in the context of pathological conditions relating to neurodegeneration, although the mechanisms by which this occurs are not fully understood.

The inventors have now shown that other cell types, such as BL cells are subject to 5-HT mediated apoptosis via SERT.

According to the invention, a compound selected from fluoxetine, paroxetine, citalopram, fluvoxamine and a non-selective serotonin re-uptake inhibitor, is for use in treating neoplasia of B-lymphocytes. The compound may be used in the manufacture of a medicament for treating neoplasia of B-lymphocytes.

In a preferred embodiment, the subject of treatment is also undergoing treatment by concomitant or sequential delivery of an agent for increasing the susceptibility of the B-lymphocytes to apoptosis, wherein said agent is paclitaxel, irinotecan, fludarabine, cyclophosphamide, docetaxel, gemtuzumab ozogamicin, cytosine arabinoside, dexamethasone or an antisense agent targeted at Bcl-2.

It will be understood that although the proposed mechanism of action is via SERT, the invention should not be construed as being limited thereto, and it is possible that the action of the agents may be SERT independent.

In normal individuals the amount of free circulating 5-HT is regulated, at least in part, by the SERT. In depressed individuals, the amount of free circulating 5-HT is too low. Drugs which block the uptake of 5-HT via the SERT either selectively (known as selective serotonin re-uptake inhibitors (SSRI's)) or non selectively help maintain an adequate level of free circulating 5-HT and are widely used in the treatment of clinical depression. As might be expected, administration of such drugs at doses used to prevent 5-HT uptake to neoplastic cells which would otherwise undergo apoptosis in the presence of 5-HT, prevents or reduces such apoptosis.

Most surprisingly, the inventors have discovered that at higher concentrations, i.e. at concentrations above those realised for alleviation of the symptoms of depression, 5-HT re-uptake inhibitors can actually drive apoptosis in cells expressing SERT.

The present invention is particularly applicable to neoplastic cells having a relatively high susceptibility to apoptosis. Such susceptibility may be due to relatively low expression of cell survival genes, such as Bcl-2. Cells of this type include Burkitt's lymphoma cells.

Alternatively, said cell may be induced to thave a relatively high susceptibility to apoptosis, hence the second aspect of the invention.

Increasing susceptibility of the neoplastic cell to apoptosis may be achieved by interfering with the replication of at least one cell survival gene, interfering with the expression (e.g. during transcription or translation) of at least one cell survival gene and/or negating or reducing the survival function of at least one expressed cell survival gene.

It is known that blocking the production of the Bcl-2 gene product can, at least *in vitro*, lead to increased effectiveness of a number of anticancer therapies such as chemotherapy, radiation and immunotherapy. Drugs on which the removal of Bcl-2 production has been shown to have a beneficial effect include; Paclitaxel (Taxol^{®}), Irinotecan (Camptosar^{®}), Fludarabine (Fludara^{®}), Cyclophosphamide (Cytoxan^{®}), Docetaxel (Taxotere^{®}), Gemtuzumab ozogamicin (Mylotarg^{®}) Cytosine arabinoside and Dexamethasone. Preferably, said agent for increasing the susceptibility of said neoplastic cell to apoptosis is an antisense drug targeted at Bcl-2, an example of which is Genasense (Trademark, Genta Inc.).

Administration (of said said pharmaceutically active agent and/or said agent for increasing the susceptibility of said neoplastic cells to apoptosis when present) may be systemic or local by any convenient route e.g. orally, subcutaneously, intravenously, or by direct administration (injection or otherwise) into the neoplastic site. In general, said treatment will preferably comprise multiple administrations whereby to maintain an effective concentration of drug in the neoplastic cells.

Said agent (or agents) may be administered in liquid form, as a tablet or capsule, as a suspension or in solution. In each case said agent(s) may be in admixture with standard excipients, binders, taste modifiers and pH adjusters as appropriate.

It will be understood that said agent for increasing the susceptibility of said neoplastic cell to apoptosis may be administered concomitantly with said active agent (either combined in a single medicament or by coadministration of separate medicaments) or prior to administration of said active agent. Where separate medicaments are administered (concomitantly or otherwise) the routes of administration may be different.

For the treatment of anxiety or depression, SSRIs are generally administered orally at a dose of 20-100 mg/day, at which doses a steady state blood level of SSRI of about 1 µM is obtained after an initial period. Preferably, when said agent is an SSRI, and particularly where said administration is systemic, it will be administered at a level to obtain a steady state blood concentration of greater than about 1µM, more preferably greater than about 5 µM and most preferably greater than about 10µM. It will be understood that the skilled person will readily be able to calculate the dosage required to achieve said steady state levels. When said administration is non-systemic, sufficient agent will preferably be administered to achieve a concentration in the neoplastic tissue of 5 µM or more, more preferably about 10 µM or more, and most preferably about 25 µM or more.

It is expected that similar dosage levels will also be effective for d-fenfluramine.

Embodiments of the invention will now be described, by way of example only, with reference to the accompanying diagrams in which:
Fig. 1 shows the effect of 5-HT concentration on DNA synthesis in L3055 BL cells,
Fig. 2 shows the correlation between plating density and 5-HT sensitivity for L3055 cells,
Fig. 3 shows the effect on DNA synthesis in various cell lines of the addition of 125µM 5-HT,
Fig. 4 shows the effect of 5-HT treatment on cell cycle status in L3055 cells,
Fig. 5 shows the effect of increasing dose of 5-HT on the percentage of dead cells in a population of L3055 cells,
Fig. 6 shows the dose dependent variation in the number of viable L3055 cells in a population in response to increasing 5-HT levels,
Fig. 7 shows a graphical representation of FACS analysis of L3055 cells treated with 5-HT showing increased caspase activity,
Fig. 8 shows the inability of various 5-HT antagonists in preventing 5-HT from inhibiting DNA synthesis in L3055 cells,
Fig. 9 shows the effect of low doses of SSRI's in preventing 5-HT causing inhibition of DNA synthesis in L3055 cells,
Fig. 10 shows the inability of pargyline to prevent DNA synthesis inhibition by 5-HT,
Fig. 11 shows the same experiment as fig 10 replacing pargyline with clorgyline or deprenyl,
Fig. 12 shows the level of oxidatively damaged DNA in L3055 cells upon treatment with 5-HT or H₂O₂,
Fig. 13 shows western blot analysis for various BL cell lines,
Fig. 14 shows that L3055 cells carry a SERT with similar properties to those previously described for the full length neuronal SERT.
Figs. 15 to 17 show the dose dependent inhibitory effect of fluoxetine, paroxetine and citalopram respectively on DNA synthesis in L3055 cells,
Fig. 18 shows the effect on normal blood cells of doses of fluoxetine which result in tumour cell killing,
Fig. 19 shows the dose dependent inhibitory effect of imipramine on DNA synthesis in L3055 cells,
Fig. 20 shows the effect on the survival rate of the lymphoma cells under increasing concentrations of fluoxetine,
Fig. 21 shows the effect of fluoxetine treatment on cell cycle status in lymphoma cells,
Fig. 22 shows the triggering of the apoptotic cascade in response to treatment with various SSRI's,
Fig. 23 shows an increase in the levels of free intracellular Ca²⁺ in response to treatment of L3055 cells with fluoxetine,
Fig. 24 shows the effect of SSRI's on cell killing in a BL cell line transfected with the pro survival gene Bcl-2,
Fig. 25 shows the triggering of the apoptotic cascade in response to treatment with imipramine, and
Fig. 26 shows the effect of imipramine on cell killing in a BL cell line transfected with the pro survival gene Bcl-2 of Fig. 24.

### EXAMPLE 1: serotonin (5-HT)

### 1. Action of 5-HT in Driving Apoptosis in Burkitt's Lymphoma Cells.

Monoamines, including 5-HT, have been reported to induce apoptosis in cultured neuronal cells, with cerebellar granule neurons being particularly sensitive: indeed, serotonin-induced neuronal cell death has been implicated as a possible cause of neurodegenerative and neuropsychiatric disorders.

The inventors have now shown that 5-HT is a rapid promoter of programmed cell death (apoptosis) in BL lines that remain faithful to the original biopsy phenotype. This action of 5-HT is mediated through an active serotonin uptake mechanism. The serotonin transporter therefore represents a novel therapeutic target in Burkitt's lymphoma.

### 1.1. Materials and Methods.

### 1.1.1. Cell lines.

Group I Burkitt's lymphoma cell lines L3055 (EBV^{neg}), BL2 (EBV^{neg}), Elijah (EBV^{pos}), and MUTU I (EBV^{pos}) were maintained in early passage as described previously (Baker MP, Eliopoulos E, Young LS, Armitage RJ, Gregory CD, Gordon J. Blood (1998); 92:2830-2843). The late passage group III BL line MUTU III (EBV^{pos}) was also used with all cells cultured in RPMI 1640 medium supplemented with 10% Serum Supreme (BioWhittaker, Wokingham, UK), 2 mM glutamine, 100 IU/ml penicillin and 100µg/ml streptomycin. Stable *bcl*-2 and *bcl*-x_{L} transfectants of L3055 cells, together with those carrying the mammalian expression vector pEF-MC1 neopA alone as controls, were generated and characterised as detailed elsewhere (Gordon J, et al. Cell Death. Diff. (2000); 7:785-794). A stable line of HEK 293 cells carrying full-length human SERT has been described previously (Qian Y, Galli A, Ramamoorthy S, Risso S, DeFelice LJ, Blakely RD. J. Neurosci. (1997);17:45-57).

### 1.1.2. Reagents.

5-HT, fluoxetine (Prozac^{®}), pargyline, clorgyline, and deprenyl were purchased from Sigma (Dorset, UK). The 5-HT receptor antagonists SDZ205-557 and methysergide were obtained from Sandoz (Basel, Switzerland) and granisetron was obtained from Smith-Kline Beecham (Harlow, Essex, UK). 5-HT binoxalate, [1,2-³H(N)-] was obtained from NEN (Zaventem, belgium). Paroxetine (Paxil^{®}) and citalopram (Celexa^{®}) were obtained from Smith-Kline Beecham and Lundbeck (Copenhagen, Denmark) respectively. Mouse monoelonal anti-human SERT Ab was purchased from Mab technologies (Stone Mountain, USA). Affinity-purified goat F(ab')₂ antibody fragment to human IgM was purchased from ICN (Ohio, USA). JC-1 (5,5',6,6'-tetrachlorol, 1',3,3'-tetraethylbenzimidazolyl-carbocyanine iodide) cationic dye was purchased from Molecular Probes (Leiden, Holland). The supersignal chemiluminescence reagent and horseradish peroxidase (HRP)-conjugated rabbit anti-mouse antibody were obtained from Pierce (Chester, UK). Primer synthesis and DNA sequencing were performed by MWG Biotech (Milton Keynes, UK). Moloney murine leukaemia virus (MMLV) reverse transcriptase and DNasel (amplification grade) were obtained from Gibco (Paisley, UK). DNTPs and Taq polymerase were obtained from Promega (Southampton, UK). RNAguard and oligo d(T)₁₂₋₁₈ were obtained from APBiotech (Bucks., UK). RNAzol reagent was obtained from Biogenesis (Poole, England). Syto 16 was obtained from Molecular Probes Europe (Leiden, The Netherlands). All other chemicals were obtained from Sigma (Poole, U K), and were of the best grade available.

### 1.1.3. Assessment of DNA synthesis.

DNA synthesis was determined by measuring ³H-thymidine ([³H]Tdr; Amersham, UK) incorporation into cellular DNA. Cells were cultured in 200 µL of supplemented medium at densities and for times indicated below in flat-bottomed, 96-well tissue culture plates (Becton Dickinson, Oxford, UK) then pulsed for the final 4 h with 50 µl of 10 µCi/ml [³H]Tdr before harvesting on a Skatron cell harvester (Helis Bio Ltd, Newmarket, U K).

### 1.1.4. Viability assays.

Changes in the viability of cells cultured under conditions indicated below were quantified by assessment of forward and 90° (side) light scatter of cells as previously described (Dive C, et al. Biochimica Biophysica Acta (1992); 1133:275-285) using an EPICS XL-MCL flow cytometer (Beckman Coulter, Miami, FL, USA). Prior to analysis, cells were harvested into polythene FACS tubes, and fixed at 4°C in 400 µl of FACS buffer [PBS with 5% NGS and 2% Formaldehyde] (BDH/MERCK). Cells were gated in two populations (viable and dead) according to their forward and side light scatter. Viability was expressed as a percentage of the total number of cells residing in the viable and non-viable gates. Cells were also analysed according to Schuurhuis et al (Schuurhuis GJ, et al. Exp. Haematol. (1997); 25:754-759) in order to assess the percentages within treated populations of those that were viable, apoptotic, or necrotic. Syto , 16 was added at a concentration of 250 nM in PBS to 500,000 cells and incubated at RT for 30 min, at which time 5 µg/ml propidium iodide (PI) was added. Samples were then analysed immediately by FACS and a 2D plot generated of syto 16 fluorescence *versus* PI fluorescence: syto 16 is taken up only by viable cells whereas PI exclusively enters necrotic cells - syto 16^{-ve}/PI^{-ve} cells are deemed apoptotic.

### 1.1.5. Cell Cycle Analysis.

Cells cultured under conditions indicated below were harvested into 100 µl of 1x PBS followed by 400 µl cell cycle buffer containing Triton X-100 and propidium iodide and left to incubate at 4°C for 1 hr to allow staining of DNA. The degree of fluorescence emitted by bound PI is directly proportional to the amount of DNA in each cell and was measured by flow cytometry as previously described (MacDonald I, et al. Blood (1996); 87:1147-1154). Results are expressed as percentage of viable cells residing in each phase of the cycle.

### 1.1.6. Measures of apoptosis.

Apoptosis was assessed by staining cells with acridine orange and visualising nuclear morphology exactly as described previously (Gordon J, et al. Cell Death. Diff. (2000); 7:785-794). Viable cells display a homogeneous chromatin-staining pattern, whereas apoptotic cells characteristically show condensed and fragmented chromatin. Mitochondrial depolarisation was assessed using the JC-1 cationic dye. Cells were incubated with 10 µM of JC-1 for 30 min before being visualised on a LSM 510 confocal microscope (Zeiss, Germany). JC-1 exhibits potential-dependent accumulation in mitochondria accompanied by a shift in fluorescence emission from 525 nm (green) to 590 nm (red). Mitochondrial depolarisation is indicated by a decrease in the red/green fluorescence ratio.

### 1.1.7. Detection of caspase activity.

Caspase activity was detected using the CaspaTag Caspase Activity Kit (Intergen, Oxford, UK). Cells cultured under the conditions indicated below were harvested into 300 µl of culture medium at a cell density of 10⁶cells/ml, followed by the addition of 5 µl of a 30x working solution of FAM-VAD-FMK (a carboxy fluorescent tagged caspase inhibitor) (Intergen Co., USA). Cells were then incubated for 1 hr at 37°C under 5% CO₂ while protecting the tubes from light. At the end of the incubation, they were washed twice in 2 ml of 1x washing buffer, followed by addition of 2 µl of PI to distinguish dead cells from live cells, then analysed on an EPICS XL-MCL flow cytometer or by confocal microscopy. For the latter, treated cells were harvested into 300 µl of culture medium containing 10 µl of FAM-VAD-FMK before incubating for 1 hr under the conditions described above. Next, 1.5 µl of Hoechst 33342 stain was added for 5 min, and cells were washed twice in 2 ml of 1x washing buffer following the addition of 1 µl of PI.

### 1.1.8. Detection of oxidative damage to DNA.

Oxidative damage to DNA was quantified using the OxyDNA assay (Biotrin, Dublin, Ireland). Following culture of cells under conditions indicated below, they were washed first in 1x PBS then in wash solution followed by addition of 100 µl of blocking solution with a 1 hr incubation at 37°C. On washing twice in working solution, cells were then incubated with 100 µl FITC-conjugate for 1 hr in the dark at RT before being washed twice in washing solution and once in PBS. Finally, cells were resuspended in FACS buffer and analysed on an EPICS XL-MCL flow cytometer.

### 1.1.9. Western Blotting.

Cell pellets were resuspended in 100 µl of lysis buffer (25 mM Tris pH 7.6; 150 mM NaCl; 1 µg/ml aprotonin; 10 µg/ml leupeptin; 1 mM PMSF; 1 mM sodium orthovanadate; 1 mM EDTA and 1 % Triton X-100). The samples were incubated for 1 hr at 4°C, pelleted at high speed for 10 minutes at 4°C, and 100 µg of protein per sample were subjected to 10 % SDS/PAGE. SERT-transfected HEK cells were used as a positive control. After running, gels were equilibrated in transfer buffer (25 mM Tris pH 8.3; 150 mM glycine; 20% (v/v) methanol) and proteins were transferred to PVDF membranes. Blots were blocked in buffer (10 mM Tris pH 7.5; 10 mM NaCl; 0.1 % Tween 20) containing 5% (w/v) milk and were probed overnight at 4°C with a 1/5000 dilution of mouse monoclonal anti-SERT antibody. Blots were developed with an HRP-conjugated anti-mouse secondary antibody and visualised using an enhanced chemiluminescence method.

### 1.1.10. 5-HT uptake assay.

Transport assays were performed by incubating 2 x 10⁷ cells with 100 nM of 5-HT plus 400 nM of ³H-5-HT for 0 to 60 minutes at 37°C in HBSS containing 1 % BSA and 10 mM HEPES (pH 7.2). The assay was stopped by five-fold dilution in ice-cold PBS. Cell pellets were resuspended in scintillation fluid, and the total counts per pellet determined. Non-specific uptake was assayed in parallel at 4°C under the same conditions. The data for non-specific uptake were subtracted from the total uptake values to give the specific uptake.

### 1.2 Results.

### 1.2.1. 5-HT inhibits DNA synthesis in group I BL lines.

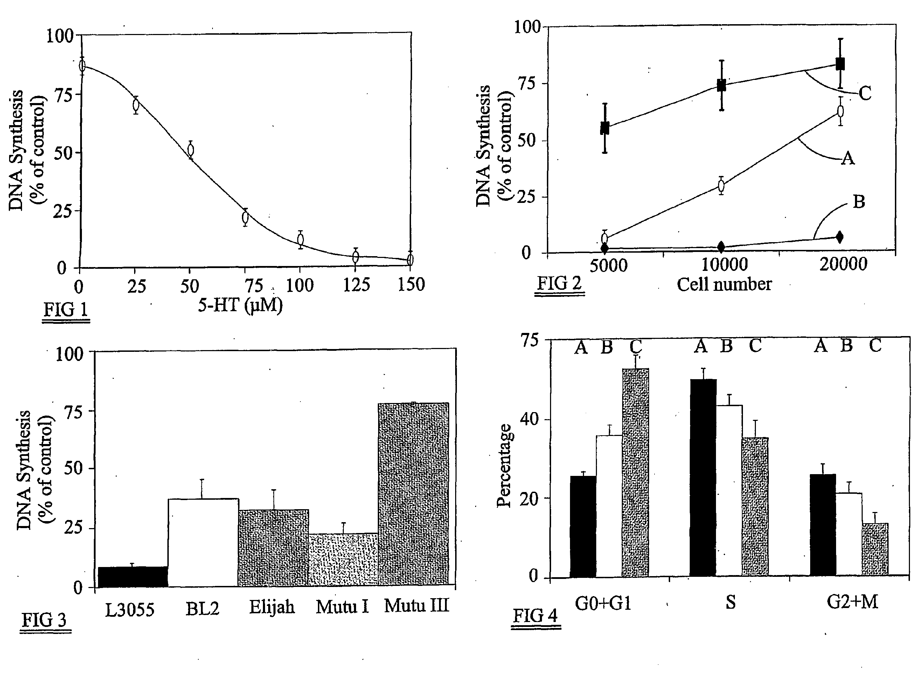

The EBV-negative L3055 line maintained in early passage has been extensively characterised and widely used as an *in vitro* model for biopsylike BL cell behaviour. In initial experiments, L3055 cells were treated with concentrations of 5-HT ranging from 1 to 150µM and their proliferative capacity assessed by [³H]-thymidine incorporation at 24, 48, and 72 hours. Maximum effects were observed after 24 hr and these data are shown in Figure 1. It can be seen that 5-HT caused a reduction in DNA synthesis in a concentration-dependent manner as evidenced by decreasing [³H]-thymidine incorporation with increasing concentrations of 5-HT.

Sensitivity to 5-HT was found to correlate with the number of cells plated: thus, whereas > 50 µM 5-HT was required to achieve 50% inhibition of DNA synthesis when cells were cultured at the relatively high plating density of 10⁵ per ml (2x10⁴/well), when plated at 2.5 x 10⁴ per ml (5x10³ per well), 50% inhibition was achieved with around 5 µM 5-HT. Likewise, Figure 2 shows that at a fixed 5-HT concentration of 50 µM (indicated by arrow A), an approximate 35%, 70%, and >90% inhibition of DNA synthesis was achieved for cells plated at 2x10⁴, 10⁴, and 5x10³ per well respectively. As would be expected, lower concentrations resulted in less inhibition (arrow C, 5 µM) and higher concentrations resulted in greater inhibition (arrow B, 150 µM). Particularly in very early passage, even 1 µM 5-HT could effect a significant reduction in the level of DNA synthesis otherwise occurring in L3055 cells at lower numbers (data not shown). This suggests that relatively low doses could be more effective if the tumour burden can be reduced, for example in combination with alternative therapies.

Three other group I BL cell lines were also investigated, namely: Mutu I, Elijah, and BL2. Figure 3 shows that when these were treated with a high concentration (125µM) of 5-HT for 24 hr of culture, a substantial decrease in DNA synthesis as measured by [³H]-thymidine incorporation was again noted. In contrast, Mutu III, an apoptosis resistant (group III) BL cell line, showed only marginally decreased DNA synthesis in response to 5-HT. The unrelated T-cell leukaemia line, Jurkat, was found to be insensitive to 5-HT actions (not shown).

It is well documented that cessation of DNA synthesis can be achieved in group I BL cells through the engagement of BCR (B cell receptors) which is accompanied by growth arrest in the G₀/G₁ compartment of the cell cycle and subsequent entry into programmed death. The inventors have shown that 5-HT similarly encouraged arrest at a distinct phase of the cell cycle. Figure 4 compares the cell cycle status of L3055 cells after 24 hr treatment with 5-HT or anti-µ chain antibody an antibody to BCR (to ligate the BCR) (columns A - control, columns B - 5-HT 150µM, columns C - anti-µ chain antibody 10µg/ml): each was used at its maximal concentration for inhibiting DNA synthesis and inducing cell death. Although BCR-ligation exerted a somewhat greater influence on the cell cycle profile, both treatments resulted in an accumulation of arresting cells in the G₀/G₁ compartment with a corresponding disappearance from both the S and G₂/M phases of cycle compared to control cultures.

### 1.2.2. 5-HT-promoted inhibition of DNA synthesis in group I BL lines is accompanied by cell death.

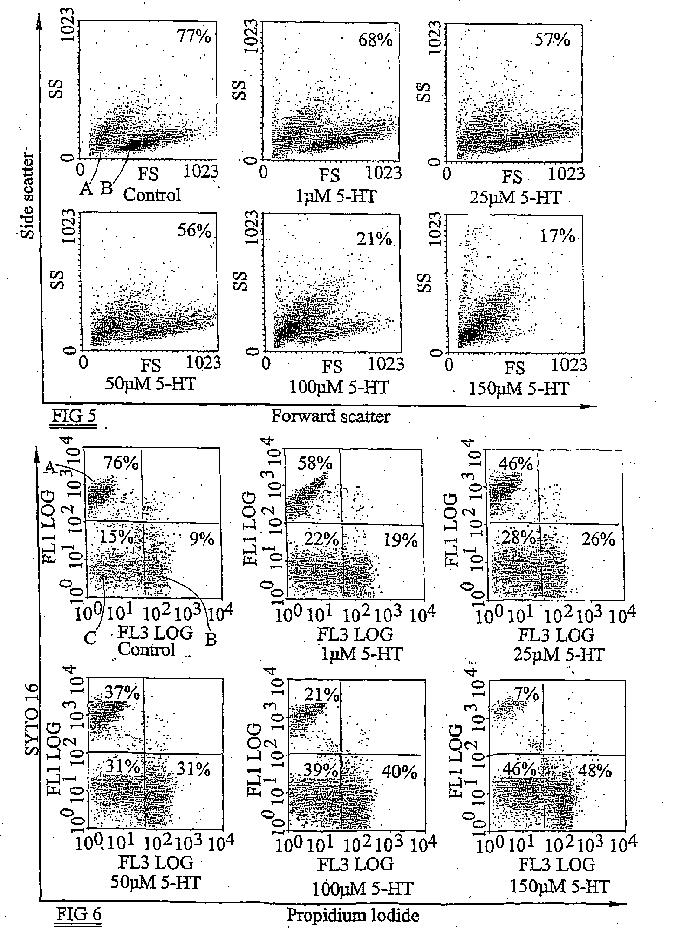

In order to ascertain whether 5-HT-promoted cessation of DNA synthesis was accompanied by cell death, L3055 cells were again treated with 1 to 150µM of 5-HT, cultured for 24 hr but then at the end of the incubations analysed for forward *versus* side scatter profile by FACS. The cells were gated into two populations based on different light scatter properties: large live cells and shrunken dead cells. As seen from Figure 5, 5-HT treatment resulted in a dramatic concentration-dependent increase in the number of L3055 cells acquiring light scatter characteristics of dead cells (indicated by arrow A) over live cells (indicated by arrow B), the percentages in the Figure representing the percentage of live cells. The background presence of 20 - 25% dead cells is typical of early passage BL lines maintaining biopsy traits (see control panel). In an analogous set of experiments (on somewhat earlier passage cells), 5-HT treated cells were double stained with syto 16 and propidium iodide (PI): syto 16 is taken up only by viable cells whereas PI only enters cells whose membranes have become permeabilised. It can be seen from Figure 6 that the number of viable cells (indicated by arrow A) (syto 16^{+ve}/PI^{-ve}) decreased in a concentration-dependent manner in response to serotonin with a trend similar to that assessed from changes in light scatter properties described above. By the end of a 24 hr exposure to 5-HT, approximately equal numbers of cells had assumed characteristics of necrosis (indicated by arrow B) (syto 16^{-ve}/PI^{+ve}) versus apoptosis (indicated by arrow C) (syto 16^{-ve}/PI^{-ve}).

### 1.2.3. Characteristics of 5-HT-promoted apoptosis in group I BL lines.

The staining of the 5-HT-treated L3055 cells with the carbocyanine liquid crystal forming probe JC-1 highlighted a loss of mitochondrial membrane potential accompanying the 5-HT-induced apoptosis of group I BL cells. Thus, following exposure to 5-HT, there is a complete disappearance of the intense red emission from JC-1 aggregates which in control cells form within mitochondria maintaining their membrane potential integrity. Instead, 5-HT-treated cells develop a diffuse green emission that results from the disseminated distribution of relatively unconcentrated JC-1 monomers (not shown).

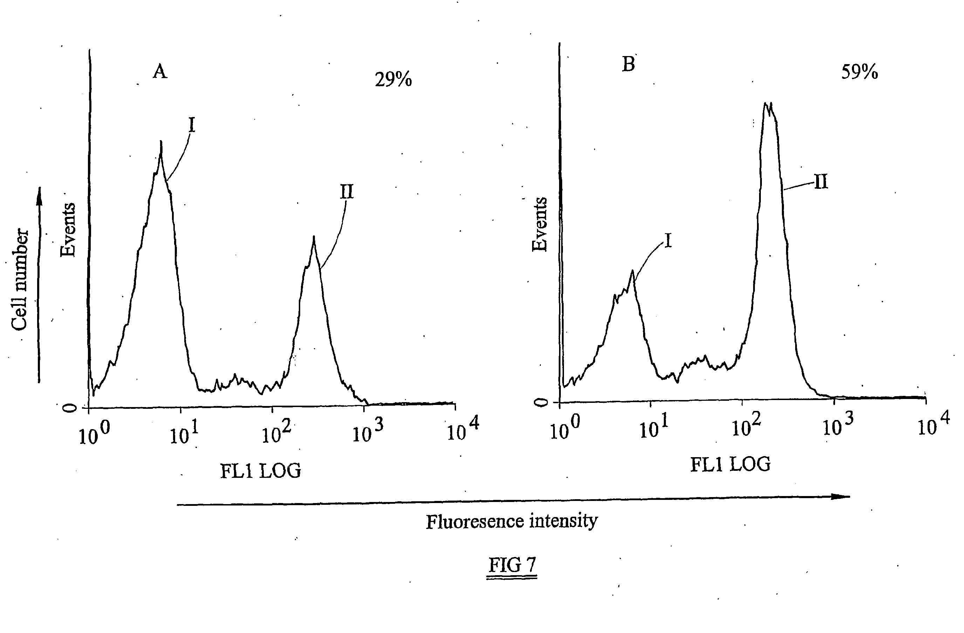

Another hallmark of programmed cell death is caspase activation although, for some B lymphocyte subsets, caspase-independent routes to apoptosis have been described. To assess whether the caspase cascade was triggered by 5-HT in L3055 cells, the carboxyfluorescent probe FAM-VAD-FMK was used. This probe binds irreversibly to caspases in their active configuration. Increased activation of caspases over background levels in L3055 group I BL cells is demonstrated by 6 hrs of treatment with 5-HT. Figure 7 illustrates this increase by FACS-based analysis. Panel A shows the relative percentage of cells staining positive (peak II) and negative (peak I) for caspase activity prior to treatment with 5-HT. Panel B shows the same data after 6 hr treatment with 5-HT. The Figure show an increase in the percentage of cells showing caspase activity from a background level of 29% to 59% after 6 hr 5-HT treatment.

### 1.2.4. Selective Serotonin Re-uptake Inhibitors inhibit actions of 5-HT on group I BL cells at relatively low levels.

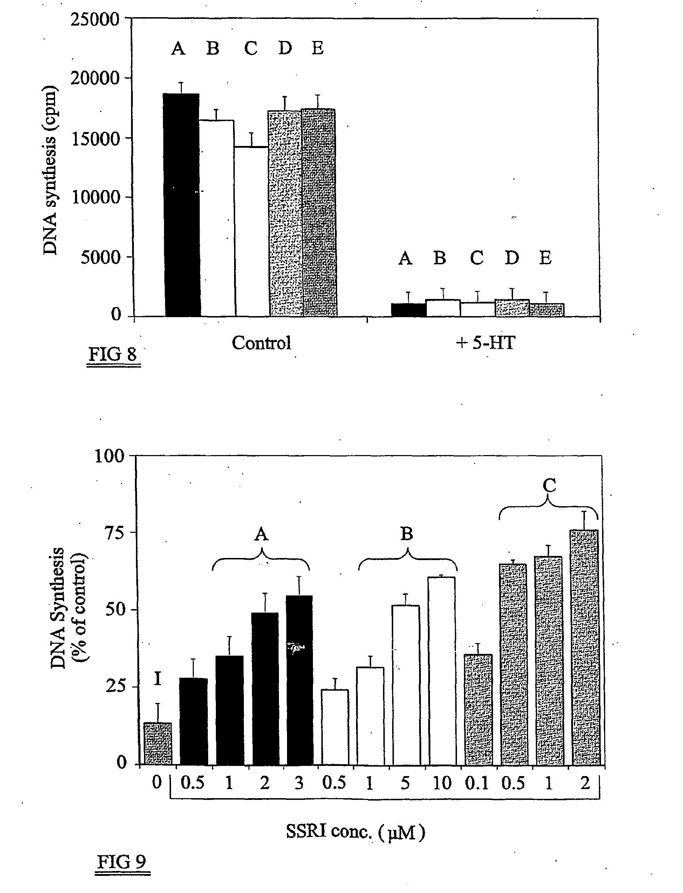

The actions of 5-HT, on both neuronal cells and lymphocytes, can be mediated either through the many different receptor subtypes to be found on the surface of the target cells or via an active uptake mechanism that relies on the serotonin transporter (SERT). To explore which of these pathways was responsible for the observed effects of 5-HT on group I BL lines, L3055 cells were pre-treated with a range of 5-HT receptor antagonists and selective serotonin re-uptake inhibitors (SSRI's). Three receptor antagonists were tested: SDZ205-557, a 5-HT₄ receptor antagonist; granisetron, a highly selective 5-HT₃ receptor antagonist; methysergide which antagonises 5-HT_{1A}, 5-HT_{1B}, 5-HT_{1D}, 5-ht_{1E}, 5-ht_{1F}, 5-HT_{2A}, 5-HT_{2B}, 5-HT_{2C}, 5-ht_{5A}, 5-ht_{5B}, 5-HT₆ and 5-HT₇ receptors. Figure 8 shows that all three receptor antagonists (column A - control (no receptor antagonist), column B - SDZ, column C - methysergide, column D - granisetron, column E - combined), at concentrations considerably higher than their affinities for the respective 5-HT receptor subtypes, and even when combined, failed to influence the ability of 5-HT to inhibit DNA synthesis in L3055 cells (compare control (no 5-HT) columns with 5-HT columns). By contrast, Figure 9 shows that three structurally distinct SSRI's, fluoxetine (columns A), citalopram (columns B), and paroxetine (columns C) each substantially reversed, in a concentration-dependent manner, the 5-HT-dependent cessation of DNA synthesis that was otherwise provoked (column I). The rank order and relative potencies for each of the SSRI inhibiting 5-HT-promoted cessation of DNA synthesis in L3055 cells correlated well with their known IC₅₀ values for blocking 5-HT uptake by neuronal SERT: fluoxetine = 6.8 nM; citalopram = 1.8 nM; paroxetine = 0.29 nM.

The actions of the SSRI's on 5-HT-dependent inhibition of DNA synthesis were reflected in their ability to reverse both 5-HT-dependent cell death and caspase activation in L3055 cells. As seen from Table 1, optimal concentrations of each of the three SSRI's were able to block substantially the loss of viability arising from a 24 hr exposure to 5-HT as judged by changes in forward *versus* side scatter properties of the cells. Similarly, 5-HT-induced caspase activation at 6 hr was almost completely abolished by pre-treatment of cells with each of the SSRI's studied.

**Table 1: Reversal of 5-HT actions by SSRI**

| **Treatments^{a}** | **% viable cells^{b}** | **% caspase activation^{c}** |
|---|---|---|
| | (24h) | (6h) |
| Control | 69.3 ± 1.20 | 27.3 ± 1.20 |
| 5-HT | 21.3 ± 8.65 | 58.3 ± 2.03 |
| Fluoxetine + 5-HT | 54.7 ± 0.88 | 30.3 ± 2.91 |
| Paroxetine + 5-HT | 58.0 ± 3.06 | 29.3 ± 4.98 |
| Citalopram + 5-HT | 55.3 ± 4.98 | 29.3 ± 1.33 |

| | | |
|---|---|---|
| ^{a}L3055 cells were cultured with or without 5-HT (150 µM) as indicated and SSRI: fluoxetine (5 µM); paroxetine (2 µM); citalopram (10 µM). ^{b}At the end of a 24 h culture period with treatments shown, cells were analysed by FACS for forward *versus* side light scatter properties as in Figure 5. The % viable cells remaining are given as means ± S.E.M. of 3 experiments. ^{c}After 6 hr of treatment, L3055 cells were stained with FAM-VAD-FMK and analysed for active caspases as for Figure 7. Results are given as % cells active caspase positive and are represented as means ± S.E.M. of 3 experiments. | | |

### 1.2.5. Evidence that 5-HT-induced apoptosis is not due to oxidative stress

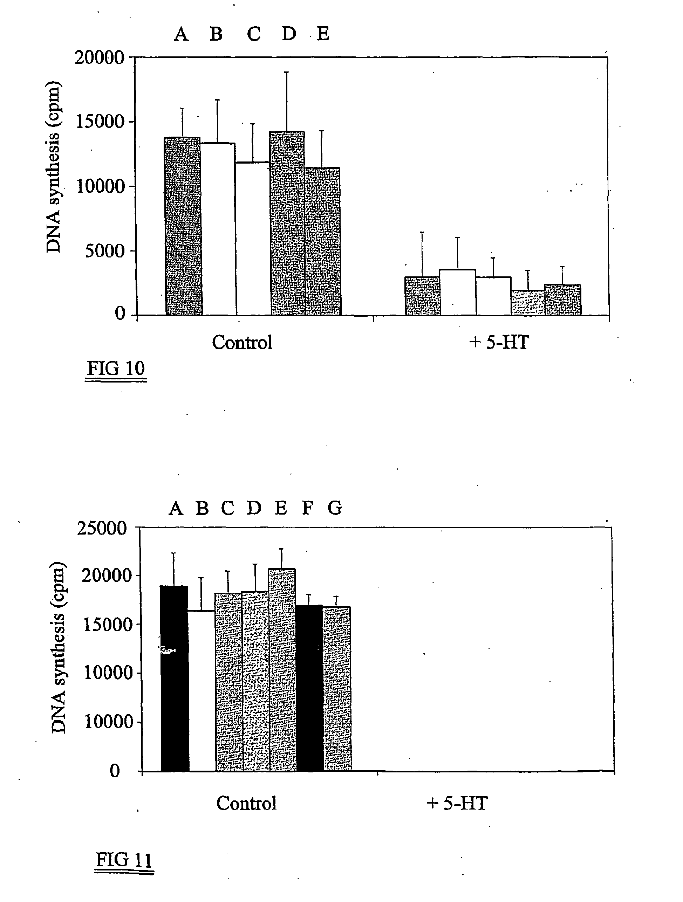

When 5-HT is transported into cells it can either be stored in vesicles until released, or converted to 5-hydroxyindoleacetic acid (5-HIAA) by the mitochondrial enzyme monoamine oxidase (MAO). This enzyme exists in two forms, MAO-A and MAO-B. As 5-HT catabolites are highly oxidative, the inventors have assessed whether, as described for some neuronal cells, 5-HT is signalling group I BL cells to undergo apoptosis by inducing oxidative stress. First, the effect of pre-treating cells with various concentrations (Figure 10: column A - 0, column B - 10µM, column C - 25µM, column D - 50µM, column E - 100µM) of the relatively broad-acting MAO inhibitor pargyline prior to adding 5-HT was investigated. As shown in Figure 10, even the highest concentration of 100 µM pargyline (column E), which had no deleterious effect by itself ("control"), failed to influence 5-HT-induced cessation of DNA synthesis ("+5-HT"). Figure 11 shows that the same outcome was observed when using clorgyline and deprenyl which, respectively, display high selectivity inhibition for the MAO-A and MAO-B isoforms (column A - 0, column B - clorgyline 10µM, column C - clorgyline 1µM, column D - deprenyl 10µM, column E - deprenyl 1µM, column F - clorgyline/deprenyl 10µM, column G - clorgyline/deprenyl 1µM.

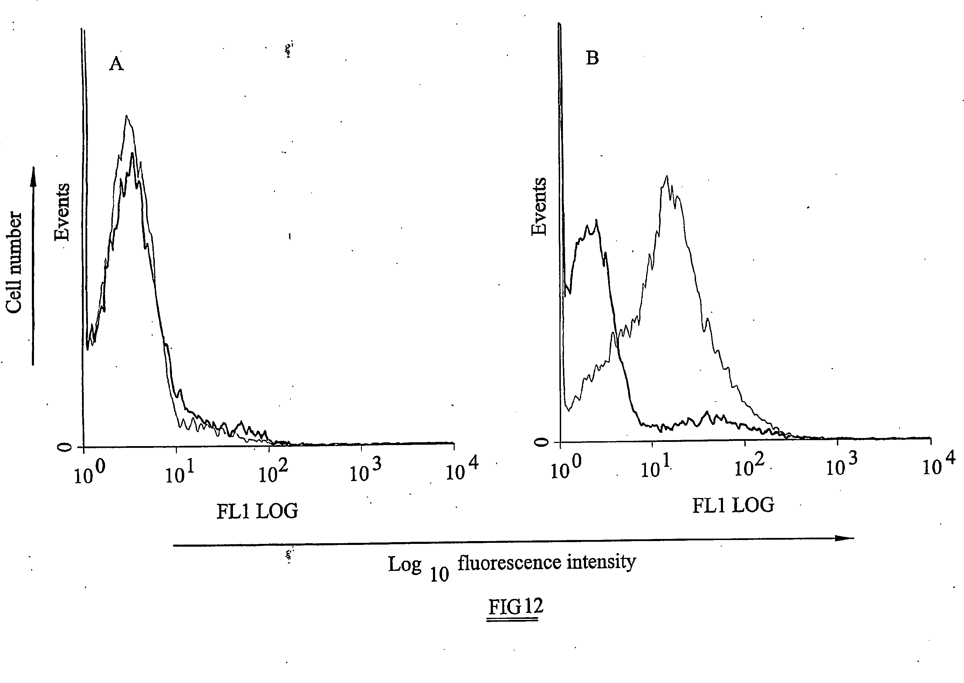

While the above experiments offer evidence that oxidative stress was not the cause of 5-HT-induced apoptosis in BL cells, further more direct examination of the generation of potential reactive oxygen species (ROS) as a consequence of 5-HT uptake was undertaken. For this the OxyuDNA assay was used, which quantifies 8-oxo-7,8-dihydro-2'-deoxyguanosine (8-oxodGuo) residues that are generated as a result of oxidative damage to DNA (see methods section 1.1.8 above). Referring to Figure 12, it can be seen that there was no increase in the level of oxidatively damaged DNA on treating L3055 cells with concentrations of 5-HT that otherwise effectively promoted apoptosis (panel A, bold plot represents cells cultured in control medium for 6 hr, faint plot represents cells cultured in medium containing 150µM 5-HT for 6 hr. Reference to panel A reveals that there was no increase in the number of cells conjugating to the fluorescent antibody). By contrast, treating cells with 30 µM H₂O₂, which also efficiently promoted apoptosis (data not shown) resulted in a large increase in the level of 8-oxoguanosine residues generated (panel B, bold plot as for Panel A, faint plot represents cells cultured in medium containing 30µM H₂O₂. Panel B shows an increase in the number of cells fluorescing, therefore indicating a greater proportion of cells containing oxidative damage).

### 1.2.6. Burkitt's lymphoma cells express a functional serotonin transporter.

The reversal of 5-HT action on group I BL cells by SSRI's indicates that 5-HT is exerting its effects via an active transport mechanism. Although the presence of SERT has been described for both EBV-transformed B lymphoblasts and normal peripheral blood lymphocytes, there have been no previous reports of its existence or characteristics on lymphoma cells of any description.

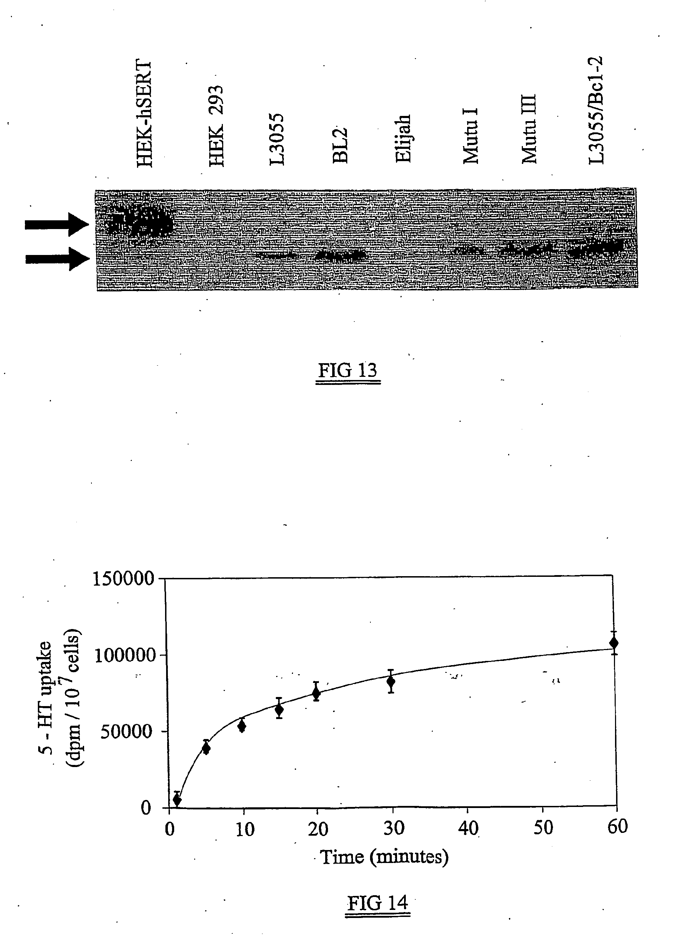

Figure 13 shows western blot analysis of cell lysates with an antibody raised to a unique sequence within SERT. This revealed the presence of protein migrating as a single band with an approximate apparent molecular weight of 70kDa in all BL lines used in this study. This band ran faster than immunoblotted protein from HEK293 cells that had been transfected with human neuronal SERT; wild-type HEK293 cells were negative for immunoreactive protein. Alternative post-translational modification of SERT in different cell types has been suggested to account for the varying size range reported for the transporter, including that of a prominent 60kDa species in some tissues. Importantly, the Mutu III line which was relatively resistant to the inhibitory actions of 5-HT (see Fig. 2) nevertheless carried equivalent levels of SERT protein to its group I counterpart, and fully resistant L3055 bcl-2-transfectants also retained high levels of SERT. Figure 13 also shows that while the group I Elijah BL cells consistently displayed a lower level of SERT protein than the other BL lines, a demonstrable immunoreactive band was always present. When assessed for first order kinetics of 5-HT uptake as shown by Figure 14, L3055 cells were shown to carry a transporter with properties similar to those previously described for full-length neuronal SERT.

### EXAMPLES 2 TO 4

### 2. Action of SSRI's in Driving Apoptosis in Burkitt's Lymphoma Cells.

### 2.1 Results.

### 2.1.1. SSRI's Inhibit DNA Synthesis and Induce Cell Death in Group I Burkitt's Lymphoma (L3055) Cell Lines.

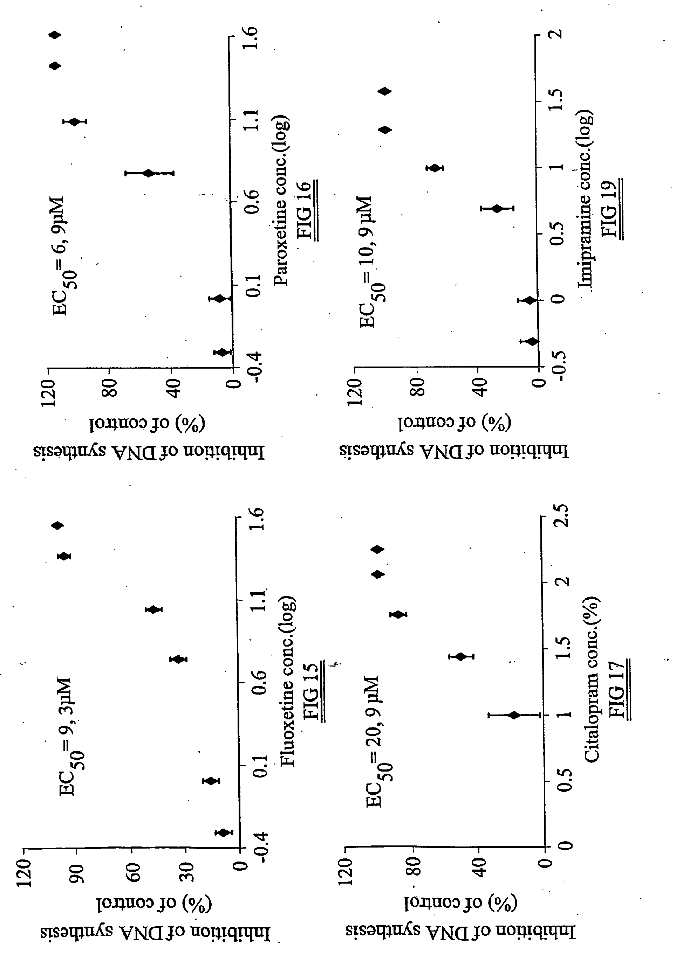

Figures 15 to 17 show the dose dependent inhibitory effect of fluoxetine (Example 2), paroxetine (Example 3) and citalopram (Example 4) respectively on DNA synthesis in the group I BL cell line (L3055). The studies used a single dose of drug (dose indicated on a log₁₀ scale) and assessed inhibition of DNA synthesis, a prelude to apoptosis, by [³H]-thymidine incorporation at 24 hrs.

The concentrations of SSRI's needed to effect killing of lymphoma cells *in vitro* were in the order of 10- to 20-fold higher than those achieved currently in the circulation of patients being administered the drugs for anti-depressant therapy.

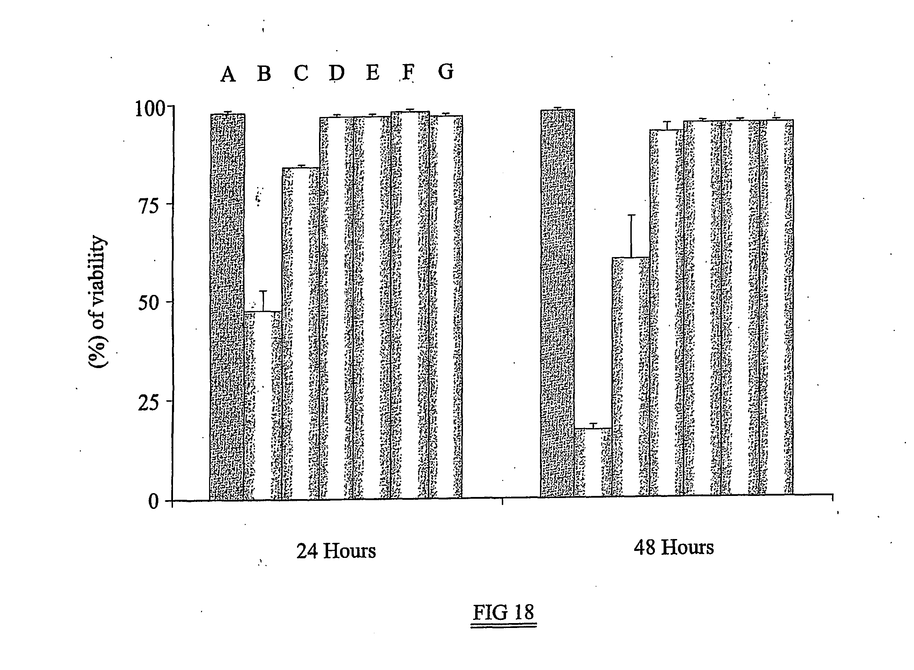

Patients on SSRI's for depression sustain their circulating levels for months on repeated administrations. Also, circulating levels are believed to represent a gross underestimation of the accumulation of SSRI in body tissues. The lymphoma are, by definition, tumours of lymphoid tissues (e.g. lymph nodes). Figure 18 shows the effect of various doses of fluoxetine on normal peripheral blood mononuclear cells (PBMC) after culture for 24 and 48 hours in media containing the drug (column A - 0, column B - 30µM, column C - 20µM, column D - 10µM, column E - 5µM column F - 1 µM, column G - 0.5µM). Importantly, this shows that normal blood cells are immune to the effects of fluoxetine at concentrations where it kills the tumour cells.

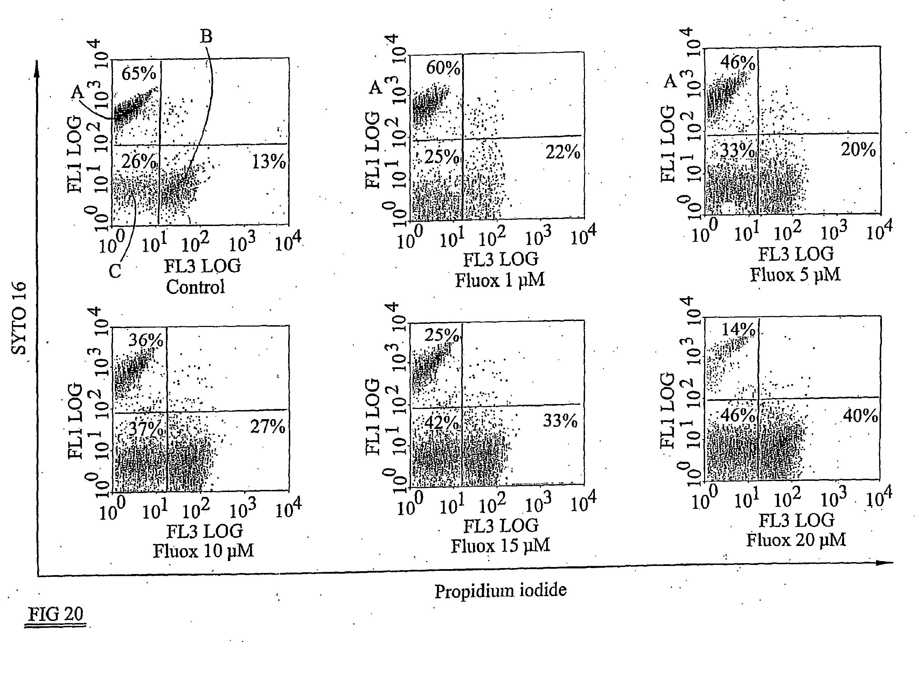

Figure 20 shows an identical experiment to that shown in Figure 6, replacing culture in media containing 5-HT with culture in media containing fluoxetine. The Figure shows that increasing the dose of the SSRI fluoxetine which was administered to populations of L3055 cells directly drove the lymphoma cells to die and again, that after 24 hrs similar numbers of cells had entered apoptosis and necrosis (quadrant A live cells, quadrant B cells entering necrosis, quadrant C cells entering apoptosis). After 24 hours a dose of 10 -15 µM fluoxetine resulted in about 40% of the lymphoma cells entering apoptosis.

### 2.1.2. Changes in Cell Cycle Profile in Response to Addition of SSRI's.

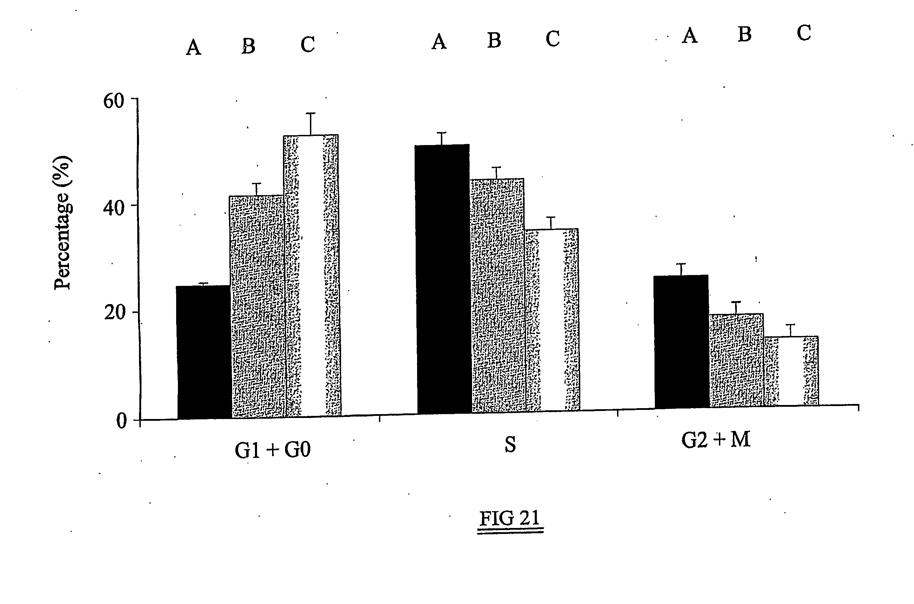

Fig. 21 compares the cell cycle status of L3055 cells after 24 hr treatment with the SSRI fluoxetine or anti-µ chain antibody (to ligate the BCR) (column A - control, column B - fluoxetine 10µM, column C - anti µ chain antibody 10µg/ml): each was used at its maximal concentration for inhibiting DNA synthesis and inducing cell death. As with the same experiment performed using 5-HT, BCR-ligation exerted a somewhat greater influence on the cell cycle profile, but both treatments resulted in an accumulation of arresting cells in the G₀/G₁ compartment with a corresponding disappearance from both the S and G₂/M phases of the cell cycle compared to control cultures. These changes are comparable with those associated with a cessation of DNA synthesis and entry into apoptosis.

### 2.1.3. Action of SSRI's in Inducing Caspase Activity and Disrupting Mitochondrial Membrane Potential.

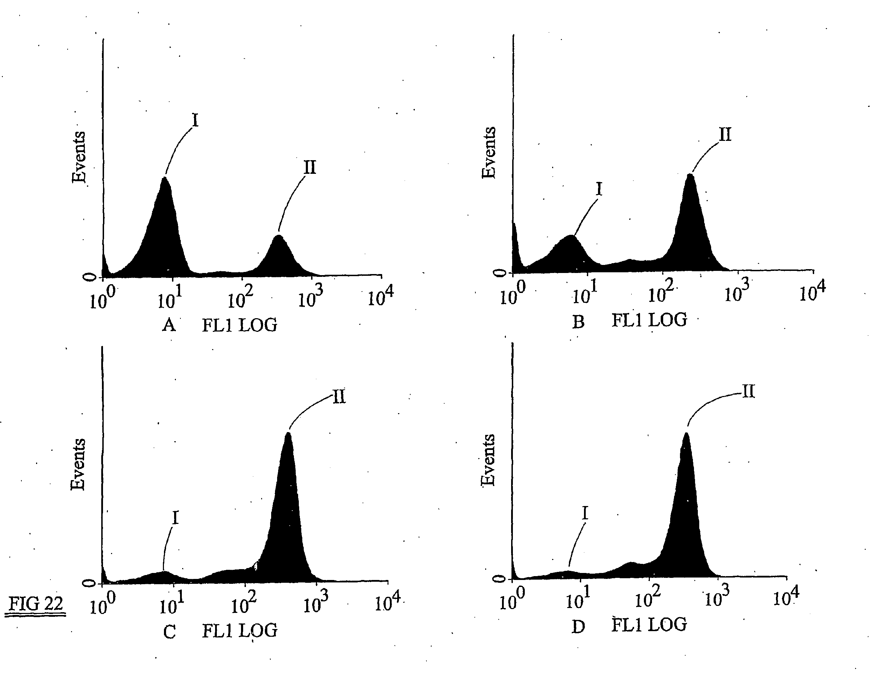

Figure 22 shows by FACS-based analysis using FAM-VAD-FMK staining that treatment of cells with SSRI's (single dose for 24 hours) triggers caspase activation (peak I number of cells staining negative for caspase activity, peak II number of cells staining positive for caspase activity) one of the key components of the apoptotic cascade with panel A being a control, panel B - fluoxetine treatment (10µM), panel C - paroxetine (10µM), and panel D - citalopram (100µM). From Figure 22, it can be seen that all of the drugs have a significant effect on the numbers of cells showing caspase activity.

A major down-stream substrate of the caspase pathway, Poly(ADP-ribose) polymerase-1 (PARP-1), undergoes cleavage in biopsylike BL cells as a result of fluoxetine action. Whereas intact PARP-1 was the almost exclusive form in control cells, 20 µM fluoxetine promoted extensive PARP-1 cleavage.

Annexin V binding to phosphatidylserine exposed on the outer leaflet of cells to signal for phagocytic engulfment is another commonly used measure of programmed death although caution should be applied when using as a sole indicator for assessing apoptosis in B cells. It was found that while untreated L3055 cells displayed low level Annexin V binding on approximately half the cells, the majority of cells developed a higher level of binding following their culture with fluoxetine (Table 2).

**Table 2. Influence of fluoxetine on phosphatidylserine exposure and DNA strand breaks in L3055 BL cells**

| **Annexin V binding^{b}** | | | |
|---|---|---|---|
| **Treatment^{a}** | | | **% TUNEL positive^{c}** |
| | **%positive** | **MFI** | |
| **Control** | 52.3 ± 1.8 | 3.9 ± 0.2 | 17.8 ± 3.7 |
| **Fluoxetine** | 81.1 ± 3.9 | 26.1 ± 8.4 | 35.7 ± 7.6 |

| | | | |
|---|---|---|---|
| ^{a}L3055 cells were cultured at 10⁶/ml for 17 h in control medium, and with Fluoxetine at 20µM. ^{b}Samples analysed by FACS to generate % positive cells within the Annexin V^{+ve}/PI^{-ve} "apoptotic" gate together with Mean Fluorescent Intensity (MFI) of stain. Results are Means ± SE of 3 separate experiments, ^{c}Samples analysed by FACS to generate % non-subdipolid cells (as assessed by PI stain) positive by TUNEL (terminal deoxynucleotidyl transferase (TdT)-mediated dUTP-biotin nick end-labelling). Results are Means ± SE of 3 separate experiments. | | | |

Neither BL cells nor their normal germinal centre B cell equivalents tended to produce the extensive "DNA ladders" that can be seen as a result of DNA fragmentation when other cells (e.g. thymocytes) undergo apoptosis. Nevertheless, whether fluoxetine might be inducing the type of DNA breaks associated with apoptosis was ascertained by using a TUNEL-based method where cells were first treated with DNA ligase before assessing the incorporation by TdT of a fluorescent-labelled nucleotide into DNA with strand breaks having blunt ends or single base overhangs. The results showed a discernible increase in the number of L3055 cells with such breaks following their treatment with fluoxetine (Table 2).

Importantly, normal peripheral blood mononuclear cells (PBMC) remained viable on exposure to SSRI. The viability of cells exposed for 24 h to 5 µM fluoxetine and to 10 µM fluoxetine was recorded as 97.3±0.88% and 97.3±0.33% respectively compared to 98.0±0.58% in control cultures. Even with 20 µM fluoxetine, viability remained at 83.7±1.20%. A near identical outcome was noted with normal resting B cells isolated from tonsils where viabilities after 24 h of culture with control medium or fluoxetine at 5, 10, or 20 µM were 97.4±0.53%, 97.0±0.41%, 96.5±0.88% and 85.9±1.07% respectively. To assess the possibility that SSRI might selectively target B cells when actively cycling, tonsilar B cells were exposed overnight to fluoxetine following 2 days of stimulation either with Staphylococcus aureus Cowan (SAC) (alone or combined with soluble CD40L) or with phorbol 12-myristate 13 acetate (PMA) (alone or in combination with ionomycin). Little inhibition of DNA synthesis occurred in any of the actively cycling populations even with fluoxetine present at 20µM. Similarly, the clear inhibitory effect of fluoxetine on DNA synthesis in biopsylike BL lines such as L3055 was not evident when assessed on 5 non-BL lines with either no, or only minor reductions, in the level of ³H-Thymidine incorporation observed in Nalm-6 ("pre-B"), RPMI 8226 ("plasmacytoid"), or the three T-cell lines: Jurkat, J10, and CEM (data not shown).

### 2.1.4. Effect of SSRI's on Intracellular Free Ca²⁺.

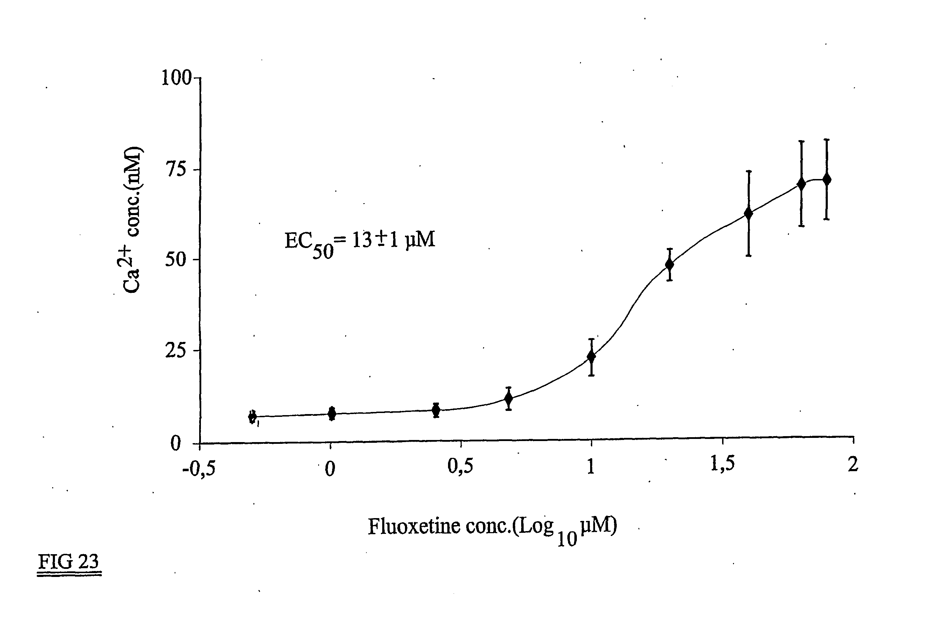

Fig 23 shows that treatment of L3055 cells with fluoxetine (indicated on a log₁₀ µM scale) leads to a sustained increase in the levels of intracellular free Ca²⁺. Fluoxetine, paroxetine and citalopram also all promoted an increase in basal Ca²⁺ with a similar shape and kinetics (data not shown). Further studies with fluoxetine have indicated that: (i) it acts on thapsigargin-sensitive endoplasmic reticulum Ca²⁺ stores and; (ii) triggers Ca²⁺ influx. The increase in intracellular free Ca²⁺ is known to be an important step in the induction of apoptosis. This further supports data suggesting that drugs which target SERT can induce apoptosis in malignant cells which do not express tumour survival genes.

### 2.1.5. Effect of the Tumour Survival Gene Bcl-2 on the Apoptotic Effect of SERT Interacting Drugs.

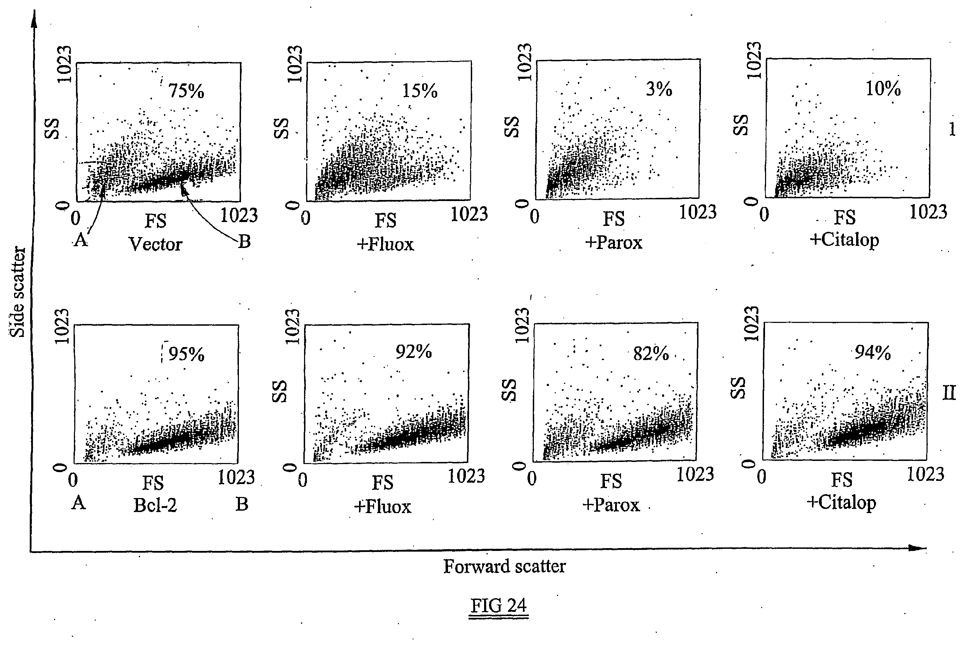

Figure 24 shows forward *versus* side scatter FACS profiles showing the effect on the survival of transfecting native BL cell lines, which are susceptible to killing by the SSRI's (SSRI concentrations are the same as those used in Fig. 22, cells were exposed to the drugs for 24 hrs). In Figure 24, arrows A indicate dead cells, arrows B indicate live cells, with the percentages given relating to the percentage of live cells. Panel I shows data for the native BL cell line and panel II for the Bcl-2 transfected cell line. As can be seen, cell killing is greatly reduced in the presence of the Bcl-2 gene product. Since this gene is expressed in most peripheral cells they will be relatively resistant to SERT interacting drug induced apoptosis.

### EXAMPLE 5: Imipramine

### 3. Action of the non-Selective Re-uptake Inhibitor Imipramine in Driving Apoptosis in BL Cells

Figure 19 shows the dose dependant effect of imipramine on DNA synthesis in group I BL cell lines (single dose, DNA synthesis measured after 24 hrs). Imipramine although not an SSRI is known to interact with SERT with a similar affinity as fluoxetine. This can be seen by the similar EC₅₀ values for DNA synthesis of the two drugs.

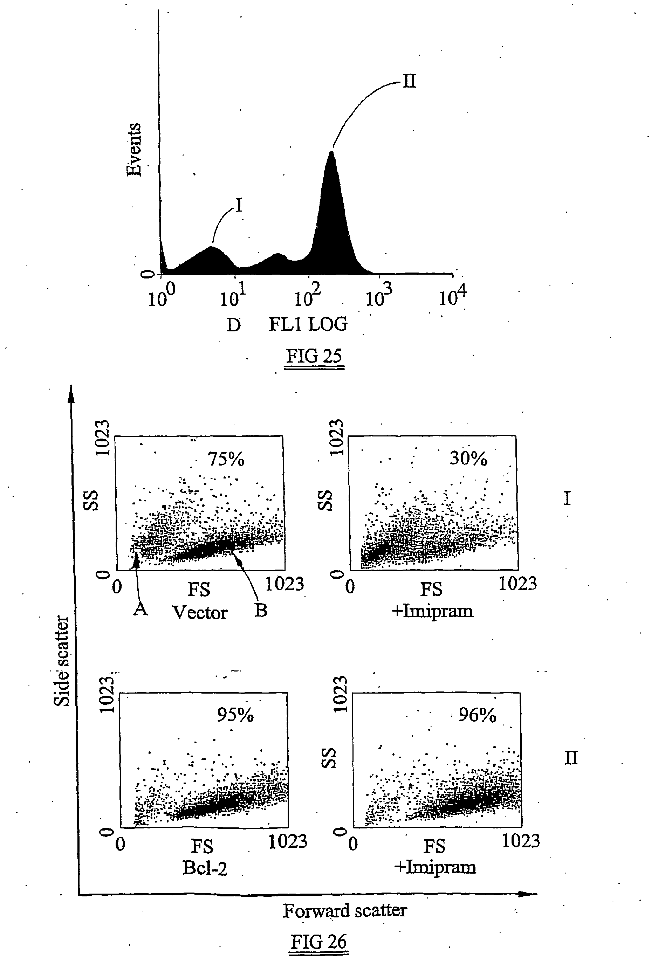

Figure 25 corresponds to Figure 22, and shows that imipramine also has a significant effect on the number of cells exhibiting caspase activity (peak I cells staining negative, peak II cells staining positive).

Referring to Figure 26, it can be seen that, as was the case for the SSRI's (figure 24), cell killing by imipramine is greatly reduced for the Bcl-2 transfected BL cell line (panel II: 91 % survival) over the native cell line (panel I: 30% survival).

### 4. Possible Role of c-myc

BL is characterised by translocations of one c-myc allele to one of the immunoglobulin loci and the extraordinarily high growth rate that characterises these tumours reflects the pro-proliferative actions of the translocated gene. Despite its hallmark translocation to immunoglobulin loci, the BL-associated constitutive expression of *c-myc* remains dependent on the binding of the recognised c-myc transcription factor Nm23-H2 to a PuF site within the regulatory sequence of the translocated gene. Using real-time RT-PCR (data not shown), it was found that a 6 h exposure of biopsylike BL cells to fluoxetine resulted in an approximate 60% reduction in the levels of both nm23-H2 and c-myc. It was also observed that there was a rapid 75% decrease in the expression of nm23-H1 and mRNA, the product of which has also been linked to c-myc expression. In contrast expression of the house-keeping gene cyclophilin A was not significantly altered with levels remaining at 81.8% (SE=8.3%) of control values following fluoxetine treatment (P=0.16).

Recently Nm23-H1 has been identified as possibly being linked to c-myc expression. Thus the SSRI appear to influence the very genes that underpin the uncontrolled cell division normally associated with BL. Furthermore, high expression of Nm23-H1 has been correlated with poor responses to treatment in high-grade lymphomas other than BL. These observations may therefore have implications for the clinical exploitation of SSRI in the broader context of B cell lymphoma.

## Claims

1. A compound selected from fluoxetine, paroxetine, citalopram, fluvoxamine and a non-selective serotonin re-uptake inhibitor, for use in treating neoplasia of B-lymphocytes.

2. A compound according to claim 1, wherein said non-selective re-uptake inhibitor is selected from imipramine, desipramine, amitriptyline and clomipramine.

3. A compound according to claim 1, which is fluoxetine.

4. A compound according to any of claims 1 to 3, wherein the neoplasia is Burkitt's lymphoma.

5. A compound according to any claims 1 to 3, wherein the neoplasia is chronic lymphocytic leukaemia.

6. Use of a compound as defined in any of claims 1 to 3, in the manufacture of a medicament for treating neoplasia of B-lymphocytes.

7. Use according to claim 6, wherein the subject of treatment is also undergoing treatment by concomitant or sequential delivery of an agent for increasing the susceptibility of the B-lymphocytes to apoptosis, wherein said agent is paclitaxel, irinotecan, fludarabine, cyclophosphamide, docetaxel, gemtuzumab ozogamicin, cytosine arabinoside, dexamethasone or an antisense agent targeted at Bcl-2.

## Patentansprüche

1. Verbindung, ausgewählt aus Fluoxetin, Paroxetin, Citalopram, Fluvoxamin und einem nicht selektiven Serotonin-Wiederaufnahmehemmer, zur Verwendung bei der Behandlung von Neoplasie von B-Lymphozyten.

2. Verbindung nach Anspruch 1, bei der der nicht selektive Wiederaufnahmehemmer ausgewählt wird aus Imipramin, Desipramin, Amitriptylin und Clomipramin.

3. Verbindung nach Anspruch 1, welche Fluoxetin ist.

4. Verbindung nach einem der Ansprüche 1 bis 3, bei dem die Neoplasie das Burkitt-Lymphom ist.

5. Verbindung nach einem der Ansprüche 1 bis 3, bei dem die Neoplasie chronische lymphatische Leukämie ist.

6. Verwendung einer Verbindung wie in den Ansprüchen 1 bis 3 definiert bei der Herstellung eines Medikaments zur Behandlung von Neoplasie von B-Lymphozyten.

7. Verwendung nach Anspruch 6, wobei auf das Subjekt der Behandlung auch eine Behandlung durch gleichzeitige oder nachfolgende Verabreichung eines Agens zur Erhöhung der Empfindlichkeit der B-Lymphozyten auf Apoptose angewandt wird, wobei das besagte Agens Paclitaxel, Irinotecan, Fludarabin, Cyclophosphamid, Docetaxel, Gemtuzumab-Ozogamicin, Cytosin-Arabinosid, Dexamethason oder ein Antisense-Agens ist, der auf Bcl-2 ausgerichtet ist.

## Revendications

1. Composé, choisi parmi la fluoxétine, la paroxétine, le citalopram, la fluvoxamine et un inhibiteur non-sélectif de la recapture de la sérotonine pour une utilisation pour un traitement de la néoplasie des lymphocytes B.

2. Composé selon la revendication 1, où l'inhibiteur non-sélectif de la recapture de la sérotonine est choisi parmi l'imipramine, la désipramine, l'amitriptyline et la clomipramine.

3. Composé selon la revendication 1, qui est de la fluoxétine.

4. Composé selon l'une des revendications 1 à 3, où la néoplasie est le lymphome de Burkitt.

5. Composé selon l'une des revendications 1 à 3, où la néoplasie est la leucémie lymphoïde chronique.

6. Utilisation d'un composé défini dans une des revendications 1 à 3 pour la fabrication d'un médicament pour le traitement de la néoplasie de lymphocytes B.

7. Utilisation selon la revendication 6, où le sujet du traitement est aussi soumis à un traitement par une administration concomitante ou séquentielle d'un agent pour une augmentation de la susceptibilité des lymphocytes B à une apoptose, où ledit agent est le paclitaxel, l'irinotécan, la fludarabine, le cyclophosphamide, le docétaxel, le gemtuzumab ozogamicin, la cytosine arabinoside, la dexaméthasone ou un agent antisense, qui est dirigé vers Bcl-2.
